# EUROPÄISCHE PATENTSCHRIFT

(11) **EP 2 579 910 B1**
(45) Veröffentlichungstag und Bekanntmachung des Hinweises auf die Patenterteilung: **10.02.2016**
(21) Anmeldenummer: 11724426.9
(22) Anmeldetag: 10.06.2011
(51) Int. Cl.: A61M 1/14, A61M 1/16, G01N 21/64

(54) **VORRICHTUNG ZUR EXTRAKORPORALEN BLUTBEHANDLUNG MIT EINER MESSEINRICHTUNG ZUR BESTIMMUNG DER LUMINESZENZ DER VERBRAUCHTEN DIALYSIERFLÜSSIGKEIT**
APPARATUS FOR EXTRACORPOREAL BLOOD TREATMENT, COMPRISING A MEASURING DEVICE FOR DETERMINING THE LUMINESCENCE OF THE SPENT DIALYSATE
DISPOSITIF DE TRAITEMENT SANGUIN EXTRACORPOREL DOTÉ D'UN DISPOSITIF DE MESURE POUR DÉTERMINER LA LUMINESCENCE DU LIQUIDE DE DIALYSE USAGÉ

(30) Priorität: 16.06.2010 EP 10006210; 11.06.2010 DE 102010023486
(43) Veröffentlichungstag der Anmeldung: 17.04.2013
(73) Patentinhaber: B. Braun Avitum AG, 34212 Melsungen (DE)
(72) Erfinder: FRORIP, Aleksandr, 61508 Elva (EE); NACKE, Christoph, 51007 Tartu (EE); MOLL, Stefan, 34212 Melsungen (DE); CASTELLARNAU, Alex, 34212 Melsungen (DE); MEIBAUM, Jörn, 34225 Baunatal (DE)
(74) Vertreter: Winter, Brandl, Fürniss, Hübner, Röss, Kaiser, Polte - Partnerschaft mbB
(86) Internationale Anmeldenummer: PCT/EP2011/059652
(87) Internationale Veröffentlichungsnummer: WO 2011/154514

(56) Entgegenhaltungen:
- EP-A1- 2 005 982
- WO-A1-99/62574
- US-A- 5 110 477
- F. ANDERS KARLSSON: "Physical-chemical properties of beta2-microglobulin", IMMUNOCHEMISTRY, Bd. 11, 3. Juli 1973 (1973-07-03), Seiten 111-114, XP002605651,

## Beschreibung

Die Erfindung bezieht sich auf eine Vorrichtung, in der eine Methode zur Bestimmung von Abfallprodukten während einer Dialysebehandlung ausgeführt wird.

Bei Patienten mit eingeschränkter oder fehlender Nierenfunktion werden Abfallprodukte des natürlichen Stoffwechsels einschließlich urämischer Toxine durch Dialyseverfahren entfernt, wobei die Entfernung der Stoffe aus dem Blut, welches extrakorporal geführt wird, durch den Kontakt des Blutes mit einer Dialysierflüssigkeit, welche mit verschiedenen Salzen versetzt ist und somit diffusive und konvektive Effekte hervorruft, welche für den Stofftransport vom Blut in die Dialysierflüssigkeit über eine Membran verantwortlich sind, erfolgt, wobei das gereinigte Blut anschließend dem Patienten wieder zu geführt wird.

Das Maß für die Dialysedosis eines Patienten kann nicht nur auf Basis der subjektiven Einschätzung der Gesundheit des Patienten erfolgen. Es ist notwendig, den Dialyseerfolg in einer Weise zu quantifizieren, so dass ein ausreichender Dialyseeffekt gewährleistet wird. Gleichzeitig ist ein zu hohes Maß an Dialyse aufgrund von Kostengründen ebenfalls zu vermeiden. Um die Dialysetherapie effizienter zu gestalten, ist es notwendig, die Dialyseeffizienz in Echtzeit zu kontrollieren, um so die Behandlung durch Anpassung der Parameter der Dialysemaschine manuell oder automatische steuern zu können.
Um eine adäquate Dialysetherapie zu gewährleisten wurde ein *Kt*/*Vᵤᵣₑₐ*-Model entwickelt, wobei Urea eines der Abfallprodukte im zu reinigenden Blut darstellt, welches zur Bestimmung einer adäquaten Dialysetherapie herangezogen wird und wobei *K* die Reinigungsleistung des Dialysators von *Urea* aus dem Blut in *ml*/*min, t* die Behandlungszeit in *min* und V das Verteilungsvolumen von *Urea* in *ml* im menschlichen Körper darstellt, welches direkt in Zusammenhang mit dem Patientengewicht steht. Der dimensionslose Faktor *Kt*/*Vᵤᵣₑₐ* definiert die Reduktion von Harnstoff-Stickstoff im Blut bei einer dreimaligen Behandlung pro Woche.

Die Bestimmung des Gehaltes von Harnstoff bzw. toxischen Substanzen im Dialysatabfluss bietet eine umfassende Überwachung des Dialyseverlaufs, allerdings müssen dazu bis heute Proben manuell dem System entnommen werden und zur chemischen Analyse an ein entsprechend ausgerüstetes Labor weitergereicht werden.

Daher wurde die kontinuierliche Überwachung der Hämodialyse gefordert *(IEEE Engineering in Medicine* & *Biology Society 11^{th} International Conference*), welche durch die Änderung der Leitfähigkeit durch die Hydrolyse von Harnstoff und/oder anderen wichtigen Molekülen in der Dialysierlösung verursacht wird. Die Kalibration von Leitfähigkeitssensoren, welche speziell für diese Applikation entwickelt wurden, ist hingegen sehr mühselig, da Einflüsse auf die Leitfähigkeit auch aus anderen Quellen entstehen können. Eine Messeinrichtung, welche nach diesem Prinzip funktioniert ist *Biostat*© *Urea Monitor* von Baxter.

Des Weiteren kann die kontinuierliche Überwachung einer Hämodialyse über optische Absorbanzmessung, welche die Transmission der Dialysierflüssigkeit, welche im Wesentlichen von Harnsäure und andere kleinmolekulare Stoffe beeinflusst wird, realisiert werden. Eine solche Messeinrichtung wird durch das *UV-Monitoring System* von Fridolin bspw. in EP 1 083 948 B1 beschrieben.

Neben der Reduktion von Harnstoff ist jedoch auch das Entfernungsverhalten von anderen Stoffen wie mittelgroßen Molekülen im Bereich von 500 bis ca. 50000 Da wichtig zur Bewertung der Dialyseeffizienz, da diese maßgeblich für die Pathologie bei einer Urämie sind. Zu ihnen zählen u.a. kleine Proteine und Peptide wie â₂-Mikroglobulin, Cystatin C, Retinol-Binding Protein (RBP) oder beispielsweise Complement Faktor D (VFD). Mit den oben beschriebenen und aus dem Stand der Technik bekannten Vorrichtungen und Verfahren ist es aber nicht möglich den Gehalt dieser Stoffe kontinuierlich während einer Dialysebehandlung im gereinigten Blut bzw. im Abfluss der Dialysierflüssigkeit zu überwachen. Eine Aussage über Moleküle mittlerer Größe wie â₂-Mikroglobulin oder anderen Peptiden bzw. kleinen Proteinen, welche maßgeblich an dem Krankheitsverlauf der Urämie verantwortlich sind, ist damit jedoch nicht möglich.

Patentschriften EP2005982 und WO 99/62574 offenbaren jeweils eine Vorrichtung nach den Merkmalen des Oberbegriffs des Anspruchs 1. Es ist daher Aufgabe der Erfindung, eine Vorrichtung zur Verfügung zu stellen, mit der kontinuierlich während der Therapie eine Aussage über den Gehalt bzw. die Änderung des Gehalts von mittelgroßen Molekülen im Bereich von 500 bis ca. 50000 Da in Abfluss der Dialysierflüssigkeit gemacht werden kann. Somit wäre ein Rückschluss auf die Reinigungsleistung der Therapie hinsichtlich dieser Moleküle bzw. die Entfernungsrate dieser Moleküle aus dem zu reinigenden Blut kontinuierlich während der Therapie also in Echtzeit möglich.

Gelöst wird diese Aufgabe durch eine Vorrichtung mit den Merkmalen des Patentanspruchs 1. Vorteilhafte Ausgestaltungen der Erfindung finden sich in den Unteransprüchen.

Absorbanz- und Lumineszenzmessungen sind analytische Verfahren zur Untersuchung von Molekülen im elektromagnetischen Spektralbereich. Lumineszenzmessungen werden üblicherweise zum Nachweis von Proteinen, welche über anregungsfähige Strukturen wie z.B. Fluorophore verfügen, eingesetzt. Neben Proteinen verfügen auch einige Peptide und andere chemische Substanzen über die Fähigkeit, elektromagnetische Strahlung aufgrund von Lumineszenzeffekten auszusenden. Solche Substanzen gelangen auch im Stoffwechselkreislauf von Säugern in deren Blut und müssen daraus entfernt werden, um eine einen auf Dauer funktionsfähigen Stoffwechsel aufrechtzuerhalten. Wenn die Nierenfunktion des Säugers beeinträchtigt ist; müssen diese Substanzen mittels einer Nierenersatzbehandlung aus dem Blut des Säugers entfernt werden.

Am Beispiel der Fluoreszenz werden spezielle Effekte der Lumineszenz beispielhaft dargelegt. Bei dem Prinzip der Fluoreszenzmessung wird eine Substanz mit monochromatischer, elektromagnetischer Strahlung einer bestimmten Wellenlänge bestrahlt. Verfügt die Substanz über Fluorophore und besitzen die eingestrahlten Photonen die molekülspezifische Energie zur Anregung von Elektronen, so werden die Photonen absorbiert, indem Elektronen von einem Grundzustand in einen höheren energetischen Zustand wechseln. Nach einer für Fluoreszenzeffekte typischen Dauer von bis zu ca. 1µs verlässt das angeregte Elektron seinen höheren energetischen Zustand und kehrt in den Grundzustand zurück. Dabei sendet es die überschüssige Energie in Form von Photonen einer bestimmten Wellenlänge aus. Diese Wellenlänge liegt üblicherweise 20-50nm oberhalb der Anregungswellenlänge und ist auf Energieverluste durch nicht-strahlende Effekte innerhalb der Energiebänder des Moleküls zurückzuführen. Dieser Unterschied in der Wellenlänge wird üblicherweise als Stokes-Shift bezeichnet ["Topics in Fluorescence Spectroscopy", Vol. 1-11, Joseph R. Lakowicz]. Gleichzeitig gibt es aber auch Stoffe, bei denen der Unterschied zwischen Anregungs- und Emissionswellenlänge wesentlich größer ist. Ein Beispiel stellt hier p-cresol dar. Die Anregungswellenlänge dieses Stoffes liegt bei ca. 300nm, während die Emissionswellenlänge bei ca. 600nm liegt. Anregungs- und Emissionswellenlänge sind charakteristische Eigenschaften für ein Molekül. Dementsprechend können über die Analyse des Absorptions- und des Emissionsspektrums bei Lumineszenzeffekten wie der Fluoreszenz Rückschlüsse auf die Substanzen innerhalb einer Flüssigkeit gezogen werden.

Die Erfindung verbindet die Methode einer Lumineszenzmessung und / oder einer Kombination aus Lumineszenz- und Absorbanzmessung mit einer Vorrichtung zur Nierenersatzbehandlung zur Bestimmung der Inhaltsstoffe von Abfallprodukten im Dialysatabfluss, wobei die Bestimmung der Konzentration einer bestimmten Substanz oder einer Kombination von Substanzen innerhalb der Dialysatabfälle direkt von der Zusammensetzung der abfließenden Dialysierflüssigkeit, welche aus dem Dialysator herausfließt, in einer Dialysebehandlung abhängt.

Der Zweck dieser Erfindung dient in erster Linie der einfachen und zuverlässigen Bestimmung von Dialysatinhaltsstoffen im Dialysatabfluss während einer Dialysebehandlung um dem Patienten eine adäquaten Behandlungserfolg zu gewährleisten, ohne diesen in zu hohem Maße zu belasten ("über-"dialysieren). Gleichzeitig ist es durch die Auswertung der Messergebnisse, welche entweder nur die Ergebnisse optischer Fluoreszenzmessungen oder die Ergebnisse aus der Kombination von optischer Fluoreszenz- und Absorbanzmessungen beinhalten, möglich, die Behandlung des Patienten an seine speziellen, individuellen Bedürfnisse anzupassen, indem dialysespezifische Parameter wie Art des Dialysators, Art der Therapieform, Höhe des Dialsatflusses usw. im Sinne des Patientenerfolges angepasst werden.

Weiter dient die Erfindung zur Realisierung einer Vorrichtung zur genauen Bestimmung von Abfallstoffen und deren Mengen im Dialysatabfluss während einer Hämodialyse. Diese Erfindung erlaubt die Kombination aus Anwendung einer bekannten Technik und die Verwendung einer zuverlässigen medizintechnischen Anlage zur Bestimmung von Proteinen/Peptiden (beispielsweise â₂-Mikroglobulin) aber auch anderen Substanzen, welche in der Dialysierflüssigkeit nach Durchgang durch den Dialysators enthalten sind, so dass durch die Messergebnisse Einfluss auf Dialyseparameter wie Dialysatorauswahl, Blutfluss, Dialysatfluss, Therapieart u.v.m. ermöglicht wird.

Bei einer mit einer erfindungsgemäßen Vorrichtung durch geführten Behandlung ist nunmehr die Bestimmung des Inhalts von Dialysierflüssigkeiten im Abfluss der Dialyse wobei die Messung des Inhaltstoffes und der Konzentration des Inhaltstoffes oder einer Kombination mehrerer Inhaltsstoffe auch im mittelmolekularen Bereich in der aus dem Dialysator herausströmenden Dialysierflüssigkeit während einer Dialysebehandlung anhand einer spektrale Untersuchung mittels Lumineszenz- bzw. Fluoreszenzmethode oder mittels Kombination aus Absorbanz- und Lumineszenz- bzw. Fluoreszenzmethode der Dialysierflüssigkeit möglich, um dialespezifische Parameter wie beispielsweise Blutfluss, Dialysatfluss, Ultrafiltrationsrate, Therapieart, Therapiedauer zu ermitteln.

Während der Therapie ist nun in Echtzeit die Bestimmung der Inhaltsstoffes bzw. der Inhaltstoffe innerhalb der im Abfluss befindlichen Dialysierflüssigkeit und / oder deren Konzentration auch im mittelmolekularen Bereich ermöglicht.

Ebenfalls ist es während der Therapie in Echtzeit ermöglicht relative Änderungen eines Inhaltstoffes oder eines Stoffgemisches im mittelmolekularen Bereich z.B. anhand eines Kt/V_{Mittelmoleküle} zu bestimmen.

Die Ergebnisse der spektralen Untersuchungen können zusammen mit den spezifischen Messparametern wie Anregungswellenlänge, Emissionswellenlänge, Intensitätsprofil der Anregungswellenlänge, und/oder Intensitätsprofil der Emissionswellenlänge dargestellt werden, um auf den Gehalts eines Stoffes oder eines Stoffgemisches in der aus dem Dialysator austretenden Dialysierflüssigkeit zurückzuschließen.

Ebenso können die Ergebnisse der spektralen Untersuchungen mit Dialyseparametern wie Dialysatfluss, Blutfluss, Dialysator etc. dargestellt werden, um auf den Gehalt eines Stoffes oder eines Stoffgemisches auf der Blutseite des Dialysators zurück zuschließen.

Dabei kann die Messung der Dialysierflüssigkeit kontinuierlich, regelmäßig oder sporadisch während einer Dialysetherapie durchgeführt werden.

Bevorzugt wird die spektralphotometrische Untersuchung mittels einer UV-Licht emittierenden Strahlungsquelle durchgeführt, da die Anregungsenergien der meisten der zu bestimmenden Substanzen im UV-Bereich liegen.

Bevorzugt werden deshalb Anregungswellenlängen für die Fluoreszenzmessung im Bereich von 1 bis 750 nm verwendet.

Weiterhin hat es sich als vorteilhaft erwiesen, dass nunmehr eine Beurteilung und Änderung bzw. Anpassung der patientenindividuellen Behandlungsparameter auf Basis der Analyse der Messergebnisse aus Fluoreszenzmessung oder der Kombination aus Fluoreszenz- und Absorbanzmessung aus vorhergehenden Therapien des gleiche Patienten und/oder des gleichen Bedienpersonals der Vorrichtung in Echtzeit vorgenommen werden kann.

Bevorzugt werden dabei als Behandlungsparameter das jeweilige Entfernungsverhalten (relative Änderungen), die Reduktionsrate und/oder die Dialysedosis Kt/V eines oder mehrerer urämischer Stoffe oder eines Stoffgemisches verwendet.

Die interessanten urämischen Stoffe sind dabei Proteine und/oder Peptide im mittelmolekularen Bereich von ∼500 bis ∼50.000 Da wie z.B. β₂-Mikroglobulin oder dgl. sowie kleinmolekulare Substanzen mit einem molekularen Gewicht bis ∼500 Da wie z.B. Harnsäure, Harnstoff oder dgl.

In einer bevorzugten Ausführung der Erfindung ist das Messergebnis bzw. das Messergebnis in Verbindung mit vorgenannten dialysespezifischen Parametern auf einer Ausgabeeinheit wie bspw. einem Bildschirm und/oder einem Drucker darstellbar.

Nach einem weiteren Gedanken der Erfindung sind ein oder mehrere Parameter für die Dialysebehandlung in Abhängigkeit von dem Messergebnis bzw. dem Messergebnis in Verbindung mit anderen dialysespezifischen anpassbar, bspw. an in einer Speichereinheit abgelegte Referenzdaten wie z. B. vorhergehenden Therapien des gleiche Patienten und/oder des gleichen Bedienpersonals.

Als besonders vorteilhaft hat sich dabei erwiesen, dass die Anpassung automatisch vorgenommen wird.

Alternativ kann die Anpassung aber auch manuell erfolgen.

Besonders vorteilhaft ist es, wenn der anzupassende Parameter der Blutfluss in der extrakorporalen Blutbahn, die Ultrafiltrationsrate, der Dialysatfluss, die Therapieart, der Dialysator und/oder die Dialysezeit ist, da dies Parameter direkten Einfluss auf die durchzuführende Therapie haben.

Um ein möglich Therapie weiter zu automatisieren, ist die erfindungsgemäße Vorrichtung dazu ausgebildet, die Anpassung einer oder mehrerer Parameter, mit welcher die Dialysetherapie vorgenommen wird, kontinuierlich in Abhängigkeit des Messergebnisses vorzunehmen.

Weitere Ziele, Vorteile, Merkmale und Anwendungsmöglichkeiten der vorliegenden Erfindung ergeben sich aus der nachfolgenden Beschreibung der Ausführungsbeispiele anhand der Zeichnungen. Dabei bilden alle beschriebenen und/oder bildlich dargestellten Merkmale für sich oder in beliebiger sinnvoller Kombination den Gegenstand der vorliegenden Erfindung, auch unabhängig von ihrer Zusammenfassung in den Ansprüchen und deren Rückbeziehung.

Es zeigen:
- Figur 1:: erfindungsgemäße Vorrichtung,
- Figur 2:: einen Intensitätsverlauf über die Wellenlänge für die Anregungs- und Emissionswellenlänge von Proteinen/Peptiden oder anderen Substanzen beispielhaft,
- Figur 3:: bevorzugter Aufbau einer Sensors zur Fluoreszenzmessung und
- Figur 4:: bevorzugter Aufbau einer Kombination aus Fluoreszenz- und Absorbanzmessung.

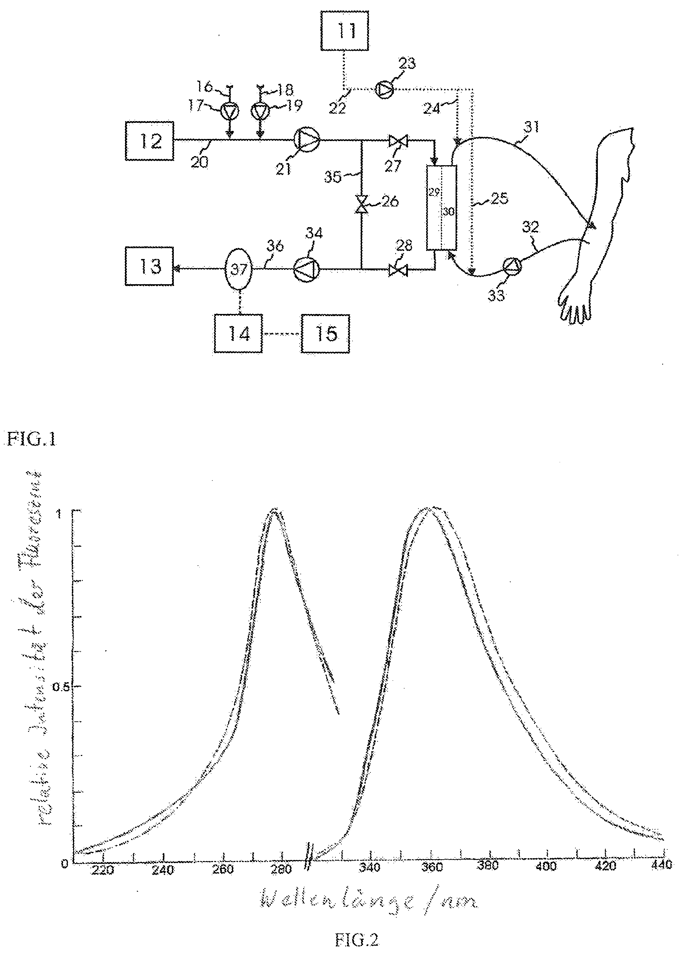

Figur 1 zeigt den Dialysatkreislauf einer konventionellen Dialysemaschine mit einer zusätzlichen Messeinheit 37, welcher im Dialysatabfluss 36 sitzt. Das Blut des Patienten wird dabei extrakorporal durch ein Schlauchsystem 32 in die blutseitige Kammer 30 eines Dialysators geführt. Nachdem das Blut den Dialysator passiert hat, wird es über das Schlauchsystem 31 dem Patienten wieder zu geführt. Der Blutfluss wird dabei durch die Blutpumpe 33 gesteuert. Die Dialysierflüssigkeit besteht aus verschiedenen Inhaltsstoffen, welche in Wasser gelöst sind. Daher verfügt die Dialysemaschine über einen Wassereinlass 12, zwei Konzentratzuführungen 16, 18 und zwei Konzentratpumpen 17, 19. Der Wasserfluss 20 bestimmt zusammen mit dem Konzentratfluss die Zusammensetzung des Dialysates. Über den Wasser bzw. Dialysatzufluss 20 wird das Dialysat der Dialysatkammer 29 des Dialysators, welche von der Blutkammer 30 durch eine semipermeable Membran getrennt ist, zugeführt. Das Dialysat wird dabei durch die Dialysatpumpe 21 dem Dialysator zugeführt und über eine weitere Pumpe 34 zusammen mit dem Ultrafiltrat dem Dialysator entzogen. Eine By- Pass-Verbindung ist zwischen den Pumpen 21 und 34 angebracht. Des Weiteren werden die Ventile 26, 27 und 28 zur Steuerung des Dialysatkreislaufes eingesetzt. Über das Schlauchsegment 36 wird das Dialysat nach dem Durchgang durch den Dialysator der Messeinheit 37, mit welcher entweder eine reine Fluoreszenzmessung oder eine Kombination aus Fluoreszenz- und Absorbanzmessung durchführbar ist, zugeführt, wo die optischen Messungen stattfinden und die Ergebnisse anschließend über ein Interface an eine zugehörige Auswerteeinrichtung 14 schickt. Anschließend werden die ausgewerteten Daten über eine Ausgabeeinheit, wie bspw. einen Bildschirm oder Drucker, ausgegeben. Nach der Messung wird das Dialysat einem Abfluss 13 zugeführt. Die gepunkteten Linien 22, 24 und 25 stellen optionale Komponenten des Systems für die Durchführung einer Hämodialfiltration dar. Dabei kommt die Substitutionsflüssigkeit aus einer entsprechenden Quelle 11 und wird über das Schlauchsystem 22 mit Hilfe einer Pumpe 23 in den Blutkreislauf des Patienten gefördert: Im Falle der Option "post dilution" wird das Substituat dem Blutkreislauf nach Durchlaufen durch den Dialysator 24 und bei der Option "pre dilution" vor dem Durchlaufen des Dialysators 25 zugeführt. Im Falle der Option "pre-post dilution" werden beide Ports 24 und 25 bei der Zuführung des Substituates verwendet. Der Computer 14 steuert alle Elemente, die in Figur 1 abgebildet sind, und einige weitere, die aus Vereinfachungsgründen weggelassen wurden. Des Weiteren bezieht der Computer 14 Informationen aus der Dialysmaschine, welche mathematisch mit den Ergebnissen der optischen Messeinrichtung verknüpft werden können.

Figur 2 zeigt einen typischen Verlauf der Intensität in Abhängigkeit von Anregungs- und Emissionswellenlänge bei einer Fluoreszenzmessung. Ausschlag der Intensität bei niedrigen Wellenlängen entspricht damit dem Intensitätsprofil der Anregungswellenlänge, während der Anstieg der Intensität bei höheren Wellenlängen das Intensitätsprofil der Emissionswellenlänge darstellt. Die Verschiebung der maximalen Intensität von Anregungs- zu Emissionswellenlänge wird Stokesshift genannt und ist auf nicht-strahlende Übergänge innerhalb des Moleküls zurückzuführen. Die durchgezogene Linie beschreibt die Spektren für feste Umgebungsbedingen (pH, T, C usw.). Durch die Änderung der Umgebungsbedingungen (pH-Wert der gelösten Flüssigkeit, Temperatur, usw.) können sich beide Spektren ändern. Dies ist durch die gestrichelte Linie angedeutet.

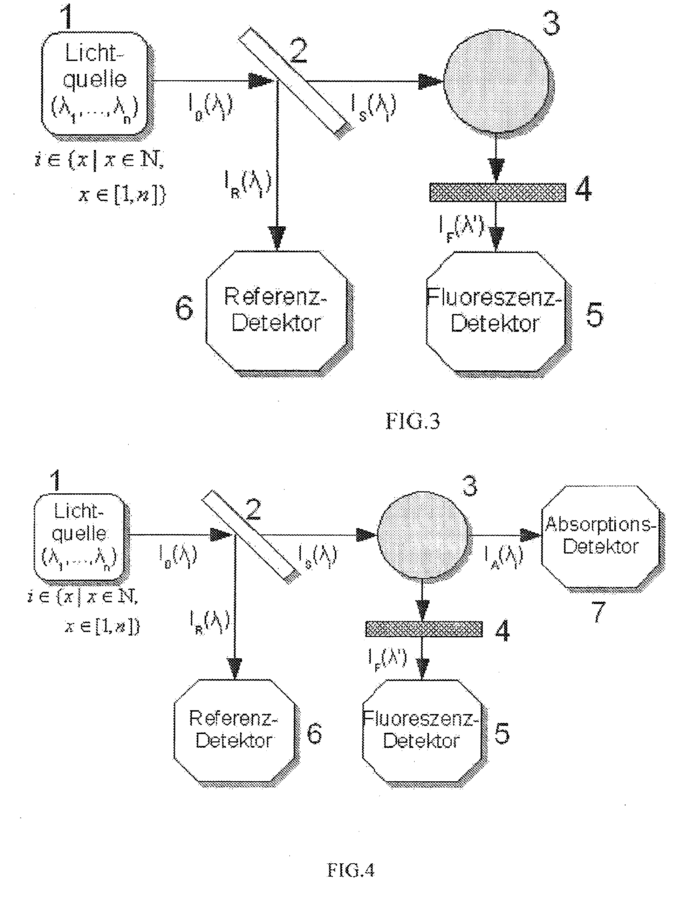

Figur 3 stellt den bevorzugten Aufbau der Messeinheit 37 mit Fluoreszenzmessung dar. Grundsätzlich kann aber auch jede Sensoreinheit für eine Lumineszenzmessung derart aufgebaut sein. Von einer Strahlungsquelle 1 ausgehend wird elektromagnetische Strahlung I0(λ) eines bestimmten Intensitäts- und Wellenlängenprofils, beispielsweise monochromatisches Licht der Wellenlänge λ =280nm, ausgesendet. Dieses wird an einem optischen Strahlengangteiler 2 geteilt. Ein Teil des Lichtes wird auf einen Referenzdetektor 6 abgelenkt und dort detektiert. Der andere Teil des Lichtes, der den Strahlengangteiler 2 ungehindert passiert, bestrahlt die zu untersuchende Probe im Ablauf 3, welche das verbrauchte Dialysat darstellt. Durch Lumineszenz- bzw. Fluoreszenzeffekte emittiertes Licht verlässt die im Abfluss 3 befindliche und wird in alle Richtungen abgestrahlt. Ein Photodetektor 5, welcher orthogonal zum ursprünglichen Strahlengang der Strahlungsquelle 1 sitzt, detektiert das durch die Probe ausgestrahlte Licht. Zur verbesserten Darstellung der emittierten Photonen sind optische Elemente 4 wie beispielsweise optische Gitter oder andere Filtereinrichtungen zwischen Ablauf 3 und Photodetektor 5 möglich, die idealerweise nur für die interessierende Wellenlänge durchlässig sind. Durch die Analyse der von der Messprobe ausgesandten Wellenlänge können Rückschlüsse auf bestimmte Inhaltsstoffe wie z.B. β2-Mikroglobulin2-Mikroglobulin oder anderen Proteinen/Peptiden und auch deren Konzentration gezogen werden.

Figur 4 beschreibt die Kombination aus Lumineszenz- bzw. Fluoreszenz- und Absorbanzmessung zur Bestimmung von Dialysatinhaltsstoffen im Abfluss 3 der Diaylsierflüssigkeit. Der Aufbau ist ähnlich dem in Figur 3, jedoch ist diese Messeinheit mit einem zusätzlichen Absorbanzdetektor 7 gekoppelt, welcher idealerweise in der optischen Achse der Strahlungsquelle 1 liegt. Die Kombination aus Absorbanzmessung bei gleichzeitiger Lumineszenz- bzw. Fluoreszenzmessung ermöglicht die Messung mittelmolekularer Stoffe wie β2-Mikrogiobulin2-Mikroglobulin bei gleichzeitiger Überprüfung der Reinigungsleistung kleinmolekularer Stoffe durch die Absorbanzmessung. Auch hier beträgt die bevorzugte Wellenlänge λ=280nm, wobei diese jedoch in einem Rahmen von 200 bis ca. 400nm variabel ist. Ansonsten ist das Prinzip der Messung stark an das Prinzip der Messung in Figur 3 angelehnt.

Neben dem bevorzugten Ausführungsbeispiel der Figuren 3 und 4 sind weitere hier nicht explizit dargestellte Ausführungsbeispiele möglich, die ebenfalls unter den Schutzbereich der beanspruchten Erfindung fallen. So zum Beispiel kann die Messeinheit 37 auch als Ein-Strahl-Fotometer ausgeführt werden. Dazu wird auf die Elemente 2 und 6 der Figuren 3 und 4 verzichtet. Ebenfalls kann die Strahlungsquelle 1 in verschiedenen Typen ausgeführten werden. So z.B. ist eine Ausführung als monochromatische oder auch als polychromatische Strahlungsquelle denkbar. Genauso kann auch die optische Filtereinrichtung 4 sowohl als Bandpass mit einem einzigen Passbereich als auch mit mehrfachem Passbereich ausgeführt werden. Durch die Variation der Strahlungsquelle 1 bzw. der Filtereinrichtung 4 können Moleküle unterschiedlicher Art detektiert werden, was eine Beurteilung der Entfernungsrate toxischer Substanzen aus dem Blut ermöglicht.

In der beschriebenen Ausführungsform gemäß den Figuren 3 und 4 wird die Messung der Dialysierflüssigkeit kontinuierlich über die Behandlungszeit durchgeführt. Allerdings ist es auch möglich, diese Messungen in diskreten Zeitintervallen durchzuführen. Die Durchführung der Messungen kann vollständig automatisiert werden, jedoch ist auch eine manuelle Aktivierung der Messung denkbar. Bei dem durchzuführenden Messverfahren handelt es sich entweder um eine reine Fluoreszenzmessung wie in Figur 2 oder um eine Kombination aus Fluoreszenz- und Absorbanzmessung gemäß Figur 3. Der Vorteil einer Kombination liegt in der Tatsache begründet, die Reduktionsrate kleinmolekularer Stoffe, welche mit der Absorbanzmessung der EP 1 083 948 B1 recht gut erfasst werden können, parallel zur Reinigungsleistung mittelmolekularer Stoffe zu bestimmen, und ins Verhältnis mit der Reinigungsleistung mittelmolekularer Stoffe, welche durch die Fluoreszenzmessung ermittelt werden kann, zu setzen. Aus dieser Kombination können Rückschlüsse nicht nur auf die aktuellen Bestandteile des Dialysates, sondern auch über Filtereigenschaften des Dialysators, Reinigungsverlauf von Molekülen unterschiedlicher Molekülgröße und Toxizität uvm. erhalten werden.

## Patentansprüche

1. Vorrichtung zur extrakorporalen Blutbehandlung mit
- einem Dialysator, der durch eine semipermeable Membran in eine erste und zweite Kammer (29, 30) geteilt ist, wobei die erste Kammer (29) in einem Dialysierflüssigkeitsweg angeordnet ist und die zweite Kammer (30) mittels einer Blutzuführleitung (32) und einer Blutabführleitung (31) mit dem Blutkreislauf eines Patienten verbindbar ist,
- einem Zulauf (20) für frische Dialysierflüssigkeit,
- einem Ablauf (30) für verbrauchte Dialysierflüssigkeit,
- einer in dem Ablauf (36) angeordneten Messeinrichtung (37), wobei die Messeinrichtung (37) wenigstens eine Strahlungsquelle (1) für im Wesentlichen monochromatische elektromagnetische Strahlung aufweist,
**dadurch gekennzeichnet, dass**
die Messeinrichtung (37) zur Bestimmung einer Lumineszenz der durch den Ablauf (36) fließenden verbrauchten Dialysierflüssigkeit wenigstens ein Detektorsystem (5) zur Detektion der Intensität der durch Lumineszenz erzeugten elektromagnetischen Strahlung aufweist.

2. Vorrichtung nach Anspruch 1, **dadurch gekennzeichnet, dass** die Strahlungsquelle (1) zur Emission elektromagnetischer Strahlung im Bereich von 1 nm bis 750 nm, insbesondere im Bereich der ultravioletten Strahlung von 1 nmm bis 380 nm ausgebildet ist.

3. Vorrichtung nach Anspruch 1 oder 2 , **dadurch gekennzeichnet, dass** das Detektorsystem (5) zur Detektion elektromagnetischer Strahlung im Bereich von 1 nm bis 750nm ausgebildet ist.

4. Vorrichtung nach einem der vorhergehenden Ansprüche, **dadurch gekennzeichnet, dass** die wenigstens eine Strahlungsquelle (1) für die im Wesentlichen monochromatische elektromagnetische Strahlung eine polychromatischen Strahlungsquelle und einen Monochromator, insbesondere einem nur für eine spezifische Wellenlänge durchlässigen optischen Filter, bzw. einen Bandpassfilter mit einem oder mehreren Passbereichen aufweist.

5. Vorrichtung nach einem der vorhergehenden Ansprüche, **dadurch gekennzeichnet, dass** das wenigstens eine Detektorsystem (5) zur Detektion der Intensität der durch Lumineszenz erzeugten elektromagnetischen Strahlung einen nur für eine spezifische Wellenlänge durchlässigen optischen Filter, bzw. einen Bandpassfilter mit einem oder mehreren Passbereichen aufweist.

6. Vorrichtung nach einem der vorhergehenden Ansprüche, **dadurch gekennzeichnet, dass** das Detektorsystem (5) als Fluoreszensdetektor ausgebildet ist.

7. Vorrichtung nach einem der vorhergehenden Ansprüche, **dadurch gekennzeichnet, dass** das Detektorsystem (5) mit seiner optischen Achse im Wesentlichen orthogonal zur optischen Achse der Strahlungsquelle (1) angeordnet ist.

8. Vorrichtung nach einem der vorhergehenden Ansprüche, **dadurch gekennzeichnet, dass** die Messeinrichtung (37) einen Referenzdetektor (6) aufweist.

9. Vorrichtung nach einem der vorhergehenden Ansprüche, **dadurch gekennzeichnet, dass** die Messeinrichtung (37) einen Strahlengangteiler (2) aufweist, der zwischen Strahlungsquelle (1) und Ablauf (36) angeordnet ist.

10. Vorrichtung nach einem der vorhergehenden Ansprüche, **dadurch gekennzeichnet, dass** die die Messeinrichtung (37) einen Absorptionsdetektor (7) aufweist, der bevorzugt in der optischen Achse der Strahlungsquelle (1) angeordnet ist, wobei sich zwischen Absorptionsdetektor (7)und Strahlungsquelle (1) in deren optischen Achse der Ablauf (36) befindet.

11. Vorrichtung nach einem der vorhergehenden Ansprüche, **dadurch gekennzeichnet, dass** die Strahlungsquelle (1) zur gepulsten oder zur kontinuierlichen Emission elektromagnetischer Strahlung ausgebildet ist.

12. Vorrichtung nach einem der vorhergehenden Ansprüche, **dadurch gekennzeichnet, dass** sie eine Mikroprozessoreinheit (14), eine Speichereinheit sowie eine Ausgabeeinheit (15) aufweist.

13. Vorrichtung nach Anspruch 12, **dadurch gekennzeichnet, dass** die Speichereinheit zur Speicherung von Referenzdaten ausgebildet ist.

14. Vorrichtung nach Anspruch 13, **dadurch gekennzeichnet, dass** die Mikroprozessoreinheit (14) zur Anpassung verschiedener Vorrichtungsparameter, wie z. B. Dialysatfluss, Blutfluss, Dialysator oder dgl., anhand eines Vergleiches der gemessenen Werte mit den Referenzdaten ausgebildet ist.

15. Vorrichtung nach einem der Ansprüche12 bis 14, **dadurch gekennzeichnet, dass** die von dem Detektorsystem (5) gelieferten Messergebnisse auf der Ausgabeeinheit (15), bspw. einem Drucker oder einem Monitor, darstellbar sind.

## Claims

1. Apparatus for extracorporeal blood treatment, comprising:
- a dialyser which is divided into a first (29) and a second chamber (30) by means of a semipermeable membrane, the first chamber (29) being arranged in a dialysate path and it being possible to connect the second chamber (30) to the blood circulation of a patient by means of a blood supply line (32) and a blood discharge line (31),
- an inlet (20) for fresh dialysate,
- an outlet (30) for spent dialysate,
- a measuring device (37) which is arranged in the outlet (36), the measuring device (37) comprising at least one radiation source (1) for substantially monochromatic electromagnetic radiation,
**characterised in that**
the measuring device (37) for determining a luminescence of the spent dialysate flowing through the outlet (36) comprises at least one detector system (5) for detecting the intensity of the electromagnetic radiation generated by luminescence.

2. Apparatus according to claim 1, **characterised in that** that the radiation source (1) is designed to emit electromagnetic radiation in the range of from 1 nm to 750 nm, in particular in the range of ultra-violet radiation of from 1 nm to 380 nm.

3. Apparatus according to either claim 1 or claim 2, **characterised in that** the detector system (5) is designed to detect electromagnetic radiation in the range of from 1 nm to 750 nm.

4. Apparatus according to any one of the preceding claims, characterised that the at least one radiation source (1) for the substantially monochromatic electromagnetic radiation comprises a polychromatic radiation source and a monochromator, in particular an optical filter transparent to only a specific wavelength or a bandpass filter having one or more pass ranges.

5. Apparatus according to any one of the preceding claims, **characterised in that** the at least one detector system (5) for detecting the intensity of the electromagnetic radiation generated by luminescence comprises an optical filter transparent to only a specific wavelength or a bandpass filter having one or more pass ranges.

6. Apparatus according to any one of the preceding claims, **characterised in that** the detector system (5) is in the form of a fluorescence detector.

7. Apparatus according to any one of the preceding claims, **characterised in that** the optical axis of the detector system (5) is arranged substantially orthogonally to the optical axis of the radiation source (1).

8. Apparatus according to any one of the preceding claims, **characterised in that** the measuring device (37) comprises a reference detector (6).

9. Apparatus according to any one of the preceding claims, **characterised in that** the measuring device (37) comprises a beam path divider (2), which is arranged between the radiation source (1) and the outlet (36).

10. Apparatus according to any one of the preceding claims, **characterised in that** the measuring device (37) comprises an absorption detector (7) which is preferably arranged in the optical axis of the radiation source (1), the outlet (36) being located between the absorption detector (7) and the radiation source (1) in the optical axis thereof.

11. Apparatus according to any one of the preceding claims, **characterised in that** the radiation source (1) is designed to emit electromagnetic radiation in a pulsed or continuous manner.

12. Apparatus according to any one of the preceding claims, **characterised in that** it comprises a microprocessor unit (14), a memory unit, and an output unit (15).

13. Apparatus according to claim 12, **characterised in that** the memory unit is designed to store reference data.

14. Apparatus according to claim 13, **characterised in that** the microprocessor unit (14) is designed to adapt various apparatus parameters, such as dialysate flow, blood flow, dialyser or the like, on the basis of a comparison of the measured values with the reference data.

15. Apparatus according to any one of claims 12 to 14, **characterised in that** the measurement results provided by the detector system (5) can be displayed on the output unit (15), e.g. a printer or a monitor.

## Revendications

1. Dispositif de traitement extracorporel de sang comprenant
- un dialyseur, qui est séparé par une membrane semi-perméable en une première et une deuxième chambre (29, 30), la première chambre (29) étant disposée dans une voie de circulation de liquide de dialyse et la deuxième chambre (30) pouvant être reliée au moyen d'un conduit d'amenée de sang (32) et d'un conduit d'évacuation de sang (31) à la circulation sanguine d'un patient,
- une arrivée (20) pour le liquide de dialyse frais,
- une évacuation (30) pour le liquide de dialyse usagé,
- un dispositif de mesure (37) disposé dans l'évacuation (36), le dispositif de mesure (37) présentant au moins une source de rayonnement (1) pour un rayonnement électromagnétique sensiblement monochromatique,
**caractérisé en ce que**
le dispositif de mesure (37) pour déterminer une luminescence du liquide de dialyse usagé s'écoulant à travers l'évacuation (36) présente au moins un système de détection (5) pour détecter l'intensité du rayonnement électromagnétique produit par la luminescence.

2. Dispositif selon la revendication 1, **caractérisé en ce que** la source de rayonnement (1) est formée pour émettre un rayonnement électromagnétique dans la plage de 1 nm à 750 nm, en particulier dans la plage du rayonnement ultraviolet de 1 nm à 380 nm.

3. Dispositif selon la revendication 1 ou 2, **caractérisé en ce que** le système de détection (5) est formé pour détecter un rayonnement électromagnétique dans la plage de 1 nm à 750 nm.

4. Dispositif selon l'une quelconque des revendications précédentes, **caractérisé en ce que** l'au moins une source de rayonnement (1) pour le rayonnement électromagnétique sensiblement monochromatique présente une source de rayonnement polychromatique et un monochromateur, en particulier un filtre optique perméable uniquement à une longueur d'onde spécifique ou un filtre passe-bande doté d'une ou plusieurs plages d'adaptation.

5. Dispositif selon l'une quelconque des revendications précédentes, **caractérisé en ce que** l'au moins un système de détection (5) pour détecter l'intensité du rayonnement électromagnétique produit par la luminescence présente un filtre optique perméable uniquement à une longueur d'onde spécifique ou un filtre passe-bande doté d'une ou plusieurs plages d'adaptation.

6. Dispositif selon l'une quelconque des revendications précédentes, **caractérisé en ce que** le système de détection (5) est formé comme détecteur de fluorescence.

7. Dispositif selon l'une quelconque des revendications précédentes, **caractérisé en ce que** le système de détection (5) est disposé avec son axe optique sensiblement orthogonal à l'axe optique de la source de rayonnement (1).

8. Dispositif selon l'une quelconque des revendications précédentes, **caractérisé en ce que** le dispositif de mesure (37) présente un détecteur de référence (6).

9. Dispositif selon l'une quelconque des revendications précédentes, **caractérisé en ce que** le dispositif de mesure (37) présente un diviseur de trajectoire de rayons (2), qui est disposé entre la source de rayonnement (1) et l'évacuation (36).

10. Dispositif selon l'une quelconque des revendications précédentes, **caractérisé en ce que** le dispositif de mesure (37) présente un détecteur d'absorption (7), qui est disposé de préférence dans l'axe optique de la source de rayonnement (1), l'évacuation (36) se situant entre le détecteur d'absorption (7) et la source de rayonnement (1) dans leur axe optique.

11. Dispositif selon l'une quelconque des revendications précédentes, **caractérisé en ce que** la source de rayonnement (1) est formée pour émettre de façon pulsée ou continue un rayonnement électromagnétique.

12. Dispositif selon l'une quelconque des revendications précédentes, **caractérisé en ce qu'**il présente une unité de microprocesseur (14), une unité de stockage, ainsi qu'une unité de sortie (15).

13. Dispositif selon la revendication 12, **caractérisé en ce que** l'unité de stockage est formée pour stocker des données de référence.

14. Dispositif selon la revendication 13, **caractérisé en ce que** l'unité de microprocesseur (14) est formée pour s'adapter à différents paramètres de dispositif, p.ex. le débit de dialysat, le débit sanguin, le dialyseur ou similaire, à l'aide d'une comparaison entre les valeurs mesurées et les données de référence.

15. Dispositif selon l'une quelconque des revendications 12 à 14, **caractérisé en ce que** les résultats de mesures fournis par le système de détection (5) peuvent être représentés sur l'unité de sortie (15), p.ex. une imprimante ou un ordinateur.
